# EUROPEAN PATENT APPLICATION

(11) **EP 2 022 561 A1**
(43) Date of publication of application: **11.02.2009**
(21) Application number: 07015389.5
(22) Date of filing: 06.08.2007
(51) Int. Cl.: B01J 20/32, B01J 45/00, B01D 15/38, C07K 1/22, C07K 14/00

(54) **Immobilisation of chelating groups for immobilised metal ion chromatography (IMAC)**

(71) Applicant: Max-Planck-Gesellschaft zur Förderung der Wissenschaften e.V., 80539 München (DE)
(72) Inventor: Görlich, Dirk, Prof. Dr., 37077 Göttingen (DE); Frey, Steffen, Dr., 37075 Göttingen-Herberhausen (DE)
(74) Representative: Weiss, Wolfgang

(57) **Abstract**

The present invention refers to a method for binding a polycarboxylic acid to a solid phase. Further, the invention refers to a solid phase having a polycarboxylic acid immobilized thereto and methods of using the solid phase, e.g. for purifying His-tagged recombinant polypeptides.

## Description

The present invention refers to a method for binding a polycarboxylic acid to a solid phase. Further, the invention refers to a solid phase having a polycarboxylic acid immobilized thereto and methods of using the solid phase, e.g. for purifying recombinant polypeptides.

A very powerful strategy for purifying recombinant proteins is the use of a "His-tag", which comprises typically 6-10 consecutive histidines. His-tag bind tightly to free coordination sites of Ni²⁺-ions. They can be released by a high concentration of imidazole, which competes for coordination sites on Ni²⁺. Such cycle of specific absorption and desorption can be used for one-step purification of a desired protein, resulting in enrichment factors of 100 or even more.

The most widely used variant of this technique uses N_{α}N_{α}-bis (carboxymethyl)-lysine coupled via the ε-amino group to agarose beads. The active group is NTA (nitrilo triacetic acid) charged with Ni²⁺, while the spacer is an amino butyl group. Such Ni²⁺-NTA-matrix has, however, several serious disadvantages, such as the high costs for N_{α} N_{α}-bis(carboxymethyl)-lysine.

An additional disadvantage is the instability of the immobilised Ni²⁺-ions. Because NTA has only 4 coordination sites for Ni²⁺, the Ni²⁺ ions leak easily from the matrix and contaminate the protein samples. This is a severe problem for at least two reasons: Ni²⁺ is a rather toxic heavy metal and it catalyses undesired oxidation of the protein sample. Furthermore, NTA-bound Ni²⁺ is easily reduced by protein-protecting agents such as DTT (dithiothreitol). Finally, metal-chelating protease inhibitors such as EGTA or EDTA cannot be combined with a Ni²⁺-NTA-matrix, because they extract the Ni²⁺-ions from this matrix.

Thus, the object according to the present invention was to provide novel methods and compositions to circumvent the problems associated with the prior art.

A first aspect of the present invention refers to a method for binding a polycarboxylic acid to a solid phase, comprising the steps: (a) providing a polycarboxylic acid and a solid phase comprising amino groups, (b) reacting the amino groups with the polycarboxylic acid in the presence of a condensing agent, wherein the condensing agent is present in a molar excess over the amino groups and the polycarboxylic acid is present in molar excess over the condensing agent and the amino groups, and wherein a single carboxyl group of the polycarboxylic acid reacts with the amino groups.

A further aspect of the present invention refers to a solid phase having a polycarboxylic acid immobilized thereto having a structure of formula (I): 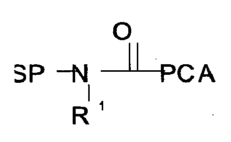 wherein SP is a solid phase;
R¹ is hydrogen or an organic residue that is compatible with the application, particularly with IMAC such as -OH, C₁-C₃ alkyl, O-C₁-C₃ alkyl, wherein alkyl may be unsubstituted or substituted, e.g. with hydroxy, carboxy and/or halogen, or a polyethylenglycol residue.

PCA is the residue of a polycarboxylic acid, particularly of an amino polycarboxylic acid, or a salt thereof.

In some embodiments of the invention, the carboxamide group -NR¹-CO- is directly bound to the solid phase. In other embodiments, the carboxamide group is bound to the solid phase via a linker, which may have a length of 1 atom up to 20 atoms, preferably 2-12 atoms, e.g. 2-6 atoms which may be selected from carbon atoms and optionally heteroatoms such as O and/or N.

Still a further aspect of the present invention is a method of purifying a recombinant polypeptide comprising the steps: (a) providing a sample comprising a polypeptide with a plurality of consecutive histidine residues, (b) contacting the sample with a solid phase as described above that contains a pre-bound complexing metal ion, e.g. a Ni²⁺ ion, under conditions wherein the recombinant polypeptide selectively binds to the solid phase, (c) separating the bound recombinant polypeptide from other sample components, and (d) eluting the recombinant polypeptide from the solid phase.

Still a further aspect of the present invention is a recombinant polypeptide comprising a "spaced histidine tag" with at least 4 histidine residues in a sequence [HₙSₘ]ₖ wherein H is histidine, S is an amino acid residue different from histidine, particularly selected from glycine and/or serine, n is in each case independently 1-4, m is in each case independently 1- 6, and k is 2-5. The spaced histidine tag may have a regular sequence, i.e. n and m have in each occurrence the same value or an an irregular sequence, i.e. n and m may have different values.

According to the present invention a method for binding a polycarboxylic acid to a solid phase is provided. The polycarboxylic acid has a plurality of carboxylic acid groups, such as 2, 3, 4, 5 or more carboxylic acid groups. The polycarboxylic acid may be an amino, nitrilo or ether polycarboxylic acid wherein amino polycarboxylic acids, particularly amino polycarboxylic acids with tertiary amino groups are preferred. Specific examples of polycarboxylic acids are ethylene diamino tetraacetic acid (EDTA), nitrilo triacetic acid (NTA), ethylene glycol tetraacetic acid (EGTA), diethylene triamino pentaacetic acid (DTPA) and triethylenetetramine-N,N,N',N',N",N" hexaacetic acid (TTHA). Especially preferred is EDTA.

The polycarboxylic acid is bound to a solid phase comprising primary or secondary amino groups. The binding is carried out under conditions which allow selective binding of only one carboxyl group of the polycarboxylic acid to the solid phase without the need of isolating an activated or derivatized form of the polycarboxylic acid such as an anhydride. By means of the reaction between carboxyl group and amino groups a carboxamide bond is formed. The solid phase may, e.g. be an amino-functionalized chromatographic support. For example, the solid phase may be selected from amino-functionalized carbohydrates such as agarose, sepharose, or cellulose, metals or semi-metals such as silicon or oxides thereof such as silica, glass, plastics such as polystyrene or lipids such as phosphatidylethanolamine- or phosphatidylserine-containing phospholipids. Further, the solid phase may comprise particles, e.g. magnetic beads. The amino groups on the solid phase are preferably primary or secondary amino groups, e.g. aliphatic primary amino groups.

The amino-functionalization of the solid phase may be carried out by known methods, e.g. by reacting an amino group-containing silane such as amino propyl triethoxysilane with a solid phase such as silica or glass or by reacting ammonia with an epoxy-activated solid phase. Preferably, the amino-functionalization of silica or glass is carried out with aminopropyl trimethoxy silane or aminopropyl triethoxy silane. Carbohydrates, such as agarose, sepharose, or cellulose are preferably first epoxy-activated e.g. with epichlorohydrine or epibromohydrine to yield epoxy-activated matrices and then treated with excess of ammonia, a primary amine, or a hydroxylamine to yield amino-modified matrices.

The amino group density on the solid phase can be varied by the reaction conditions of the amino-functionalization, e.g. temperature, duration and/or concentration of reactants. For example, the amino-functionalizing agent may be diluted with a passivating agent, e.g. a non-amino group containing silane, in order to reduce the amino group density on the surface, if necessary. The amino group containing reactant may e.g. be diluted to a concentration of 1-50% with a passivating agent. Alternatively, the amino group density may be increased by using di- or polyfunctional agents, which contain 2 or more primary or secondary amino groups such as 1,13-diamino-4,7,10-trioxatridexane. Thereby, the affinity of the matrix for His-tagged proteins may be increased. Thus, the reaction conditions may be chosen to obtain a final product with desired characteristics, e.g. such that His-tagged proteins bind specifically to the final product, while background-binding of other proteins is minimal. The optimal amino group density may be determined empirically for each solid support according to procedures described in the Examples.

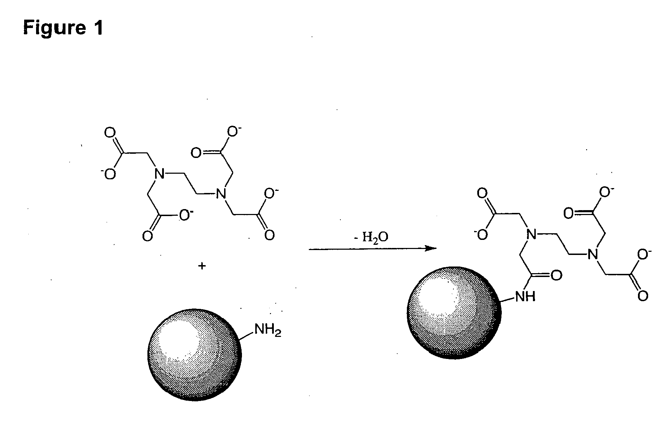

In the next step, a condensation reaction between one carboxyl group of the polycarboxylic acid and an amino group on the surface is carried out. Figure 1 shows a schematic depiction of this reaction exemplified with EDTA as polycarboxylic acid.

The reaction is carried out in the presence of a condensing agent, which may be selected from carbodiimides, carbonates such as di(N-succinimidyl) carbonate, O-(N-succinimidyl)-N,N,N',N'-tetramethyluroniumtetrafluoroborate or analogous compounds. Carbodiimides such as 1-ethyl-3-(3-dimethylaminopropyl)carbodiimide (EDC), or N,N'-dicyclohexylcarbodiimide (DCC) are preferred. Surprisingly, it was found that a single carboxyl group of the polycarboxylic acid reacts with the amino groups on the solid phase when the polycarboxylic acid is present in a sufficient molar excess over the amino groups and/or the condensing agent. In preferred embodiment, to obtain a highly specific matrix for IMAC, reaction conditions are selected wherein the amino groups are reacted substantially quantitatively with the polycarboxylic acid. Therefore, an at least 2-fold molar excess, preferably an at least 4-fold molar excess, of the condensing agent and an at least 5-fold excess, preferably an at least 10-fold molar excess and more preferably an at least 25-fold molar excess, of the polycarboxylic acid over amino groups should be used (see Examples).

The reaction is preferably carried out in an aqueous phase. The reaction conditions are preferably pH 5-9, more preferably pH 7-8.5. The polycarboxylic acid is preferably used close to the saturation limit (e.g. 0.5 M for EDTA and 0.1 M for EGTA) and the condensing agent at 1/10- 1/50 of the concentration of the polycarboxylic acid.

The reaction product is a solid phase having a polycarboxylic acid immobilized thereto via a stable carboxylic acid amide bond. Preferably, the solid phase has the structure of immobilized formula (I) as indicated above. Preferably, the immobilized polycarboxylic acid residue has still at least 2, 3, 4 or carboxyl groups and optionally further chelating groups such as amino or ether groups.

If the polycarboxylic acid is EDTA the solid phase preferably has a structure of formula (II): 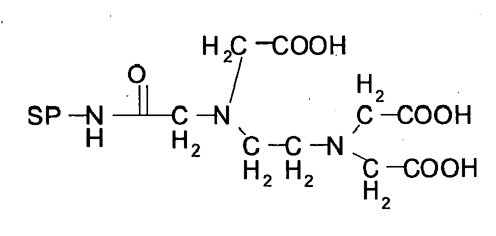 wherein SP is the solid phase.

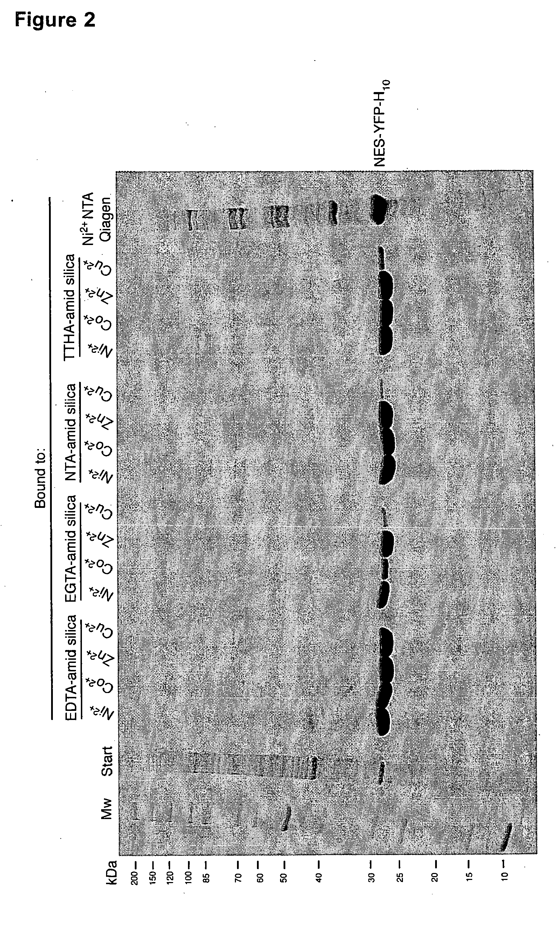

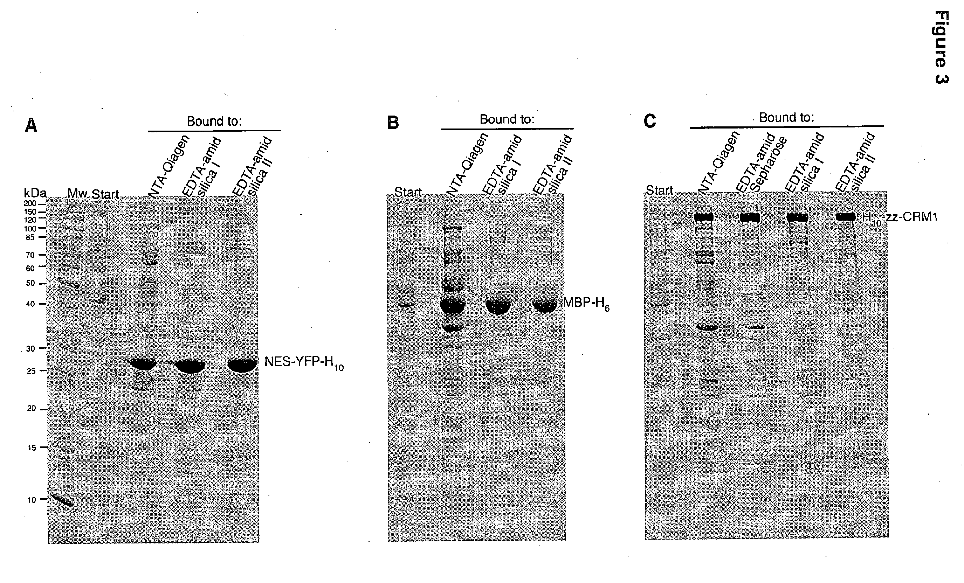

The solid phase of the invention, e.g. the EDTA-amide solid phase has a high affinity for polyvalent metal ions, such as transition metal ions, e.g. Ni²⁺, Zn²⁺, Co²⁺, or Cu²⁺-ions. Several combinations of these metal ions with chelators immobilized according to this invention provide highly selective matrices for binding histidine-tagged proteins, with less non-specific binding than the traditionally immobilised NTA-group (see Figures 2 and 3).

Thus, the solid phase of the invention may be used for immobilized metal ion chromatography (IMAC). In this chromatographic method polypeptides comprising a plurality of consecutive histidine residues, e.g. at least six consecutive histidine residues are selectively bound to a metal complex solid phase of the present invention and separated from other components. The polypeptide may be eluted from the solid phase by adding a suitable elution agent such as imidazole. The imidazole concentration is preferably in the range from 1 to 1000 mM, depending on the density of immobilized active groups, the length of the poly-histidine tag, and the multimeric state of the tagged protein.

For IMAC purification of His-tagged proteins to work, it was so far assumed that the immobilized chelator should occupy only 3-5 out of the 6 coordination sites of Ni²⁺. It is therefore a surprising aspect of this invention that immobilised hexadentate chelators such as EDTA-amide or EDTA-hydroxamide and even chelators with more than 6 coordinating groups work extremely well for this application. Apparently, histidine side chains can displace weakly coordinating groups within a given chelator. Thus, according to an embodiment of the invention, the immobilized chelator, i.e. the immobilized polycarboxylic acid amide has 6 or more coordination groups, particularly selected from amino, carboxyl, carboxamide, and hydroxamate groups.

On the solid phase of the invention, e.g. EDTA-amide, kinetically very stable complexes e.g. with Ni²⁺ may be formed. The resulting matrices are extremely resistant to Ni²⁺-extraction, e.g. by EGTA. Even 0.5 M EDTA extracts Ni²⁺ only very slowly. The resistance towards Ni²⁺-extraction increases further by including imidazole, e.g. 1 mM imidazole in the buffer. Therefore, IMAC with a solid phase of the invention, e.g. an EDTA-amide matrix, can be performed in the presence of a chelating metalloprotease-inhibitor, e.g. EDTA in concentrations of 1-10 mM, e.g. 5 mM, and/or in the presence of a thiol reagent in concentrations of 1-50 mM, e.g. 10 mM dithiothreitol (as protein-protecting reducing agent).

In an especially preferred embodiment of the present invention the chromatography is carried out with a recombinant polypeptide comprising a plurality of histidine residues in a "spaced histidine tag", preferably in a sequence [HₙSₘ]ₖ wherein H is histidine, S a spacer residue such as glycine or serine, n is 1-4, m is 1- 6, preferably 1-4, and k is 2-6. The present invention also comprises irregularily-spaced histidine tags, where the lengths of the oligo histidine clusters and the lengths of the spacer regions vary within a given tag sequence. The spaced histidine tag is preferably located at the N- and/or the C-terminus of the recombinant polypeptide.

Further, the invention is explained in more detail by the following Figures and Examples.

### Figure Legends

**Figure 1****:** Scheme for the coupling of EDTA to an amine-containing support. The reaction is carried out under conditions where a selective reaction occurs between a single carboxyl group of EDTA with the amino group on the support.
**Figure 2****:** Comparison of various Ni²⁺-containing chromatographic supports in IMAC.
E. coli cells were resuspended in buffer (50 mM Tris/ HCl pH 7.5, 2 mM magnesium acetate, 50 mM NaCl, 5 mM mercaptoethanol), a lysate was prepared and cleared by ultracentrifugation. Then, either 1 µM of a fusion protein ("NES-YFP-H₁₀", panel A) comprising a nuclear export signal (NES), the yellow fluorescent protein (YFP) and a C-terminal deca His-tag, or 1 µM of a fusion between the maltose binding protein (MBP) with a C-terminal hexa His-tag ("MBP-H₆", panel B), or 0.5 µM of a fusion protein containing a deca His-tag, a zz-tag and the mouse exportin CRM ("H₁₀-zz-CRM1", panel C) were added and used as starting materials for the binding experiments. For purification of the deca-His-tagged proteins, starting materials were supplemented with 1 mM imidazole.
400 µl starting material each were rotated o/n at 4°C with 10 µl chromatographic support. After washing with 5 ml buffer, bound proteins were eluted with 0.4 M imidazole pH 7.5. Analysis was by SDS-PAGE on 12% acrylamide gel, followed by Coomassie-staining. The load corresponds to 0.5 µl matrix.
The following chromatographic supports were used:

| | |
|---|---|
| "NTA-Qiagen": | Ni²⁺-NTA Agarose purchased from Qiagen |
| "EDTA-amide silica I": | Ni²⁺-charged EDTA-amide silica with 20% coupling density (Example 4) |
| "EDTA-amide silica II": | Ni²⁺-charged EDTA-amide silica with 5% coupling density (Example 4) |
| "EDTA-amide Sepharose ": | Ni²⁺-charged EDTA-amide Sepharose 4B (Example 1) |

**Figure 3****:** Combination of various chelators and transition metal ions for IMAC
Amino-silica with 5 % coupling density (as described in Example 4) was conjugated with either EDTA, EGTA, NTA, or TTHA. Each of the resulting matrices was then charged with either Ni²⁺, Co²⁺, Zn²⁺, or Cu²⁺. The binding assays for NES-YFP-H₁₀ were performed as described in Figure 2, however, 100 mM NaCl were included in the wash buffer.

### Examples

### Example 1: Preparation of Ni-EDTA-amide Sepharose 4B

1 liter Sepharose 4B is prewashed on a glass-funnel with 1 liter 0.1 M NaOH (in water), followed by 5 times one liter of pure water, transferred to a 5 liter flask, and filled up with water to a volume of 2 liters. 0.8 mol of NaOH are added, the temperature adjusted to 25°C, followed by addition of 1.0 mol epibromhydrine. The mixture is then shaken for 2 hours at 25°C, and then chilled on ice. The resulting epoxy-activated Sepharose is subsequently recovered by filtration through a glass funnel, washed with water, and resuspended in 2 M NH₄Cl (final concentration in water, final volume 2 Liters). 4 mol NH₃ are added from a 25% aqueous solution and the mixture is shaken o/n at room temperature.

The resulting NH₂-Sepharose 4B is recovered by filtration, washed with water until free NH₃ has be become undetectable, and resuspended in 0.5 M EDTA/Na⁺ pH 8.0 (final concentration, final volume 2 liters). Then, 50 mmol EDC are added and the mixture is shaken for 1 hour at room temperature. Thereafter, another 50 mmol EDC aliquot is added and the reaction is allowed to proceed o/n.

The resulting EDTA-amide Sepharose is recovered by filtration and washed until the free EDTA-concentration has dropped below 1 mM. The Sepharose is then charged with 20 mM NiCl₂ in water, until free Ni²⁺ appears in the non-bound fraction. Free Ni²⁺ is then removed by washing with water, 20 mM imidazole/HCl pH 7.5, 20 mM imidazole + 10 mM EDTA pH 7-8, and water, and is finally resuspended in 30% ethanol + 1 mM imidazole/HCl pH 7.5 for long-term storage.

The properties of the Ni²⁺ EDTA-amide Sepharose can be adjusted by varying the epoxy-activation step. Coupling at higher temperature (up to 40° C) and using higher concentrations of epichlorhydrine and NaOH (up to 1.2 M epichlorhydrine and 1.0 M NaOH, respectively) will result in higher coupling-density, but also in higher background-binding. Lower temperature (down to 18°C) using a lower concentration of epichlorhydrine and NaOH (down to 0.2 M epichlorhydrine and 0.1 M NaOH, respectively) will result in lower coupling density, and in even lower background binding, but also in lower specific binding capacity, in particular for proteins with short His-tags.

### Example 2: Preparation of EDTA-amide magnetic beads.

5 ml of amine-terminated magnetic beads (Sigma # 17643-5ml) are washed in water and resuspended in a final volume of 5 ml in 0.5 M EDTA/Na+ pH 8.0. A 125 µmol EDC aliquot is added and the reaction is shaken for 1 hour at room temperature. Thereafter, another 125 µmol EDC aliquot is added and the reaction is continued o/n.

The resulting EDTA-amid magnetic beads are recovered by magnetic separation, washed with water, and charged with Ni²⁺ or another metal ion analogously to Example 1.

### Example 3: Preparation of high-density EDTA-amide silica

100 g Davisil XWP1000Å 90-130 (Grace) are resuspended in 500 ml of 3% (v/v) aminopropyl triethoxy silane, 4% water, 93% ethanol and shaken for 2 hours at 50°C and then o/n at room temperature: The amino-modified silica is recovered by filtration and free silane is removed by washing with pure water. Covalent coupling to EDTA and charging with Ni²⁺ are performed as described for Sepharose 4B. For long term-storage, the product can be dried out of water or isopropanol.

### Example 4: Preparation of EDTA-amide silica with a passivated surface

The Ni²⁺ EDTA-amide silica from Example 3 three still suffers from high background-binding when used in IMAC. This background may probably result from residual silanol- and amino groups (which for sterical reasons could not react with the activated EDTA) and/or from high surface concentrations of EDTA-amide. The background-problem can be solved by including a passivating silane during the modification of silica with aminopropyl-silane. The so far best passivating silane is the reaction product between glycidyl oxypropyl trimethoxy silane and 3-mercapto 1,2-propandiol.

3 ml glycidyl oxypropyl trimethoxy silane are mixed with 3 ml 2-mercapto 1,3-propandiol, 90 ml methanol, 4 ml water and 10 µl 4-methyl morpholine, and the reaction is allowed to proceed for 30 minutes at 25°C. 150 µl aminopropyl trimethoxy silane are added, and the resulting mixture is used to modify 30 g Davisil XWP1000Å 90-130 as described above. The ratio between the aminosilane and the passivating silane is in this example defined as 5%. It can, however, be varied between 1% and 50%. The resulting passivated amino-silica is then reacted with EDTA or another chelating group and charged with metal ions as described above.

## Claims

1. A method for binding a polycarboxylic acid to a solid phase, comprising the steps:
(a) providing a polycarboxylic acid and a solid phase comprising amino groups,
(b) reacting the amino groups with the polycarboxylic acid in the presence of a condensing agent, wherein the condensing agent is present in a molar excess over the amino groups and the polycarboxylic acid is present in a molar excess over the condensing agent and amino groups, and wherein a single carboxyl group of the polycarboxylic acid reacts with the amino groups.

2. The method of claims 1, wherein the polycarboxylic acid has 2, 3, 4, 5 or more carboxylic acid groups.

3. The method of claim 1 or 2, wherein the polycarboxylic acid is an amino polycarboxylic acid.

4. The method of claim 2 or 3, wherein the polycarboxylic acid is selected from ethylene diamino tetraacetic acid (EDTA), nitrilo triacetic acid (NTA), ethylene glycol tetraacetic acid (EGTA), diethylene triamino pentaacetic acid (DPTA), and triethylene tetramine-N,N,N',N', N",N"-hexa-acetic acid (TTHA).

5. The method of any one of claim 1 to 4, wherein the solid phase is an amino-functionalized chromatographic support.

6. The method of any one of claim 1 to 5, wherein the solid phase is selected from amino-functionalized carbohydrates such as agarose, sepharose, or cellulose, metals or semi-metals such as silicon, or metal oxides or semi-metal oxides such as silica, glass, plastics such as polystyrene or lipids such as phospholipids.

7. The method of any one of claim 1 to 6, wherein the solid phase comprises particles, e.g. magnetic beads.

8. The method of any one of claim 1 to 7, wherein the amino groups on the solid phase are primary or secondary amino groups, e.g. aliphatic primary amino groups.

9. The method of any one of claim 1 to 8, wherein the condensing agent is selected from carbodiimides such as 1-ethyl-3-(3-dimethylaminopropyl) carbodiimide (EDC), or N, N'-dicyclohexylcarbodiimide (DCC).

10. The method of any one of claim 1 to 9, wherein the molar excess of the condensing agent over the amino groups is at least 2-fold, preferably at least 4-fold.

11. The method of any one of claim 1 to 10 wherein the molar excess of the polycarboxylic acid over the amino groups is at least 5-fold, preferably at least 25-fold.

12. A solid phase having a polycarboxylic acid immobilized thereto having a structure of formula (I): 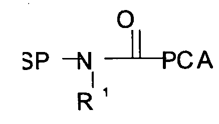 wherein SP is a solid phase;
R¹ is hydrogen or an organic residue that does not interfere with the application of the solid phase and
PCA is the residue of a polycarboxylic acid, particularly of an amino polycarboxylic acid, or a salt thereof.

13. The solid phase of claim 12, wherein PCA is the residue of EDTA or a salt thereof.

14. The solid phase of claim 12 or 13 having a structure of formula (II): 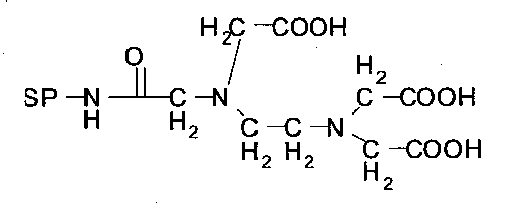 wherein SP is a solid phase;
or a salt thereof.

15. The solid phase of any of claim 12 to 14, wherein the polycarboxylic residue is complexed with a metal ion, e.g. a transition metal ion, such as a Ni²⁺ ion.

16. Use of a solid phase of any one of claims 13 to 16 for immobilized metal ion chromatography.

17. A method of purifying a recombinant polypeptide comprising the steps:
(a) providing a sample comprising a polypeptide with a plurality of consecutive histidine residues,
(b) contacting the sample with a solid phase of any one of claims 12 to 15 that contains a pre-bound complexing metal ion, e.g. a Ni²⁺ ion, under conditions wherein the recombinant polypeptide selectively binds to the solid phase,
(c) separating the bound recombinant polypeptide from other sample components, and
(d) eluting the recombinant polypeptide from the solid phase.

18. The method of claim 17, wherein the polypeptide comprises at least 6 consecutive histidine residues.

19. The method of claim 17, wherein the polypeptide comprises at least 4 histidine residues in a sequence [HₙS ₘ]ₖ wherein S is a spacer amino acid residue, n is in each case independently 1-4, m is in each case independently 1-6, and k is 2-6.

20. A recombinant polypeptide comprising at least 4 histidine residues in a sequence [HₙSₘ]ₖ wherein n is in each case independently 1-4, m is in each case independently 1-6, and k is 2-6.

21. The polypeptide of claim 20, wherein the polyhistidine sequence is at the N- and/or at the C-terminus.
